# EUROPEAN PATENT SPECIFICATION

(11) **EP 4 591 842 B1**
(45) Date of publication and mention of the grant of the patent: **15.07.2026**
(21) Application number: 25150283.7
(22) Date of filing: 06.01.2025
(51) Int. Cl.: A61F 13/0203, A61F 13/05

(54) **NEGATIVE PRESSURE WOUND DRESSING**
UNTERDRUCKWUNDVERBAND
PANSEMENT POUR PLAIES PAR PRESSION NÉGATIVE

(30) Priority: 24.01.2024 US 202418421375
(43) Date of publication of application: 30.07.2025
(73) Proprietor: DeRoyal Industries, Inc., Powell, TN 37849 (US)
(72) Inventor: Valentine, Ethan Edward, Knoxville, 37931 (US); Desai, Dhanvin Sunil, Knoxville, 37932 (US); Poker, Nicholas John, Knoxville, 37917 (US); Lawhorn, Thomas Paul, Sevierville, 37862 (US); Carter, Casey Young, Knoxville, 37919 (US)
(74) Representative: Boult Wade Tennant LLP

(56) References cited:
- US-A1- 2016 144 084
- US-A1- 2021 170 066
- US-B2- 11 007 083

## Description

### FIELD

The present disclosure relates to medical wound dressings. More particularly, the disclosure relates to improved negative pressure wound dressings configured to help avoid blistering of skin associated with many conventional negative pressure wound dressings.

### BACKGROUND

Improvement is desired in the provision of negative pressure wound dressings and particularly such dressings for incision sites. What is desired is a dressing that is of simple construction and configured to reduce blistering of skin at locations where a drape portion of the dressing steps down to the skin.

The present disclosure advantageously provides dressings constructed to have an extended skin contact layer that helps to reduce blistering of skin at locations where the drape portion of the dressing steps down to the skin.
US 2021 170066 A1 discloses, according to its abstract, a dressing which includes a drape, a pressure-sensitive acrylic-based adhesive on a skin-facing surface of the drape, an island of absorbent material, a silicone gel backing film, and a silicone gel on the silicone gel backing film.

### SUMMARY

The disclosure advantageously provides negative pressure wound dressings.

In one aspect, a negative pressure wound dressing according to the disclosure includes an island having a fluid permeable non-adhesive skin contact layer connected to a fluid absorbent layer. The absorbent layer has peripheral edges and the skin contact layer has peripheral edges extending past the peripheral edges of the absorbent layer a distance. The skin contact is positionable onto skin of a patient surrounding a wound.

A negative pressure drape overlies the island and has an adhesive lower surface and peripheral edges extending past the peripheral edges of the skin contact layer. A suction layer is connected to the negative pressure drape and in fluid communication with a source of negative pressure and the absorbent layer to enable negative pressure to be applied through the absorbent layer to the wound.

The drape steps down from the peripheral edges of the absorbent layer at a first step down and the drape steps down from the peripheral edges of the skin contact layer at a
second step down spaced outward from the first step down. The second step down has a height that is shorter than that of the first step down,. The drape is adhesively securable to the skin of the patient surrounding the wound at a juncture of the negative pressure drape and the peripheral edges of the skin contact layer defined by the second step down to provide a drape/skin juncture to enable negative pressure to be applied to the wound via the dressing. The second step down reduces damage to the skin at the drape/skin juncture during application of negative pressure to the wound.

### BRIEF DESCRIPTION OF THE DRAWINGS

Further advantages of the disclosure are apparent by reference to the detailed description when considered in conjunction with the figures, which are not to scale so as to more clearly show the details, wherein like reference numbers indicate like elements throughout the several views, and wherein:
FIG. 1 is a perspective and partial cross-sectional view showing a negative pressure wound dressing according to the disclosure.
FIGS. 2A-2D show installation of the negative pressure wound dressing.

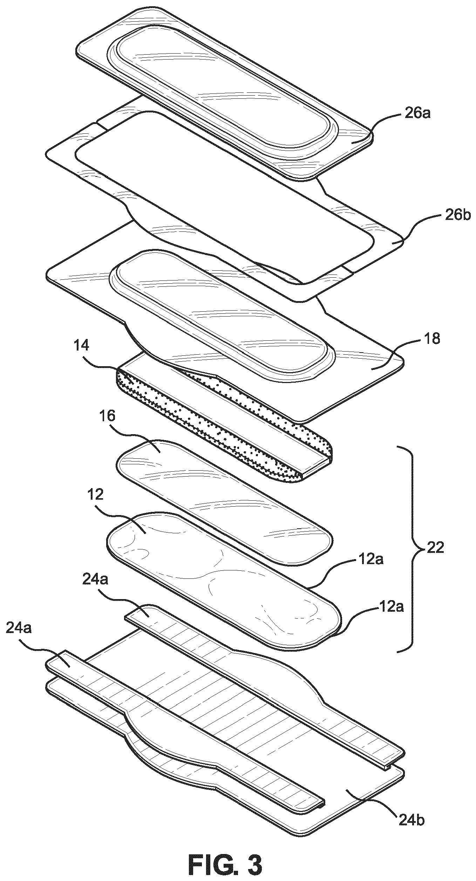

FIG. 3 is an exploded view of the negative pressure wound dressing according to the disclosure.

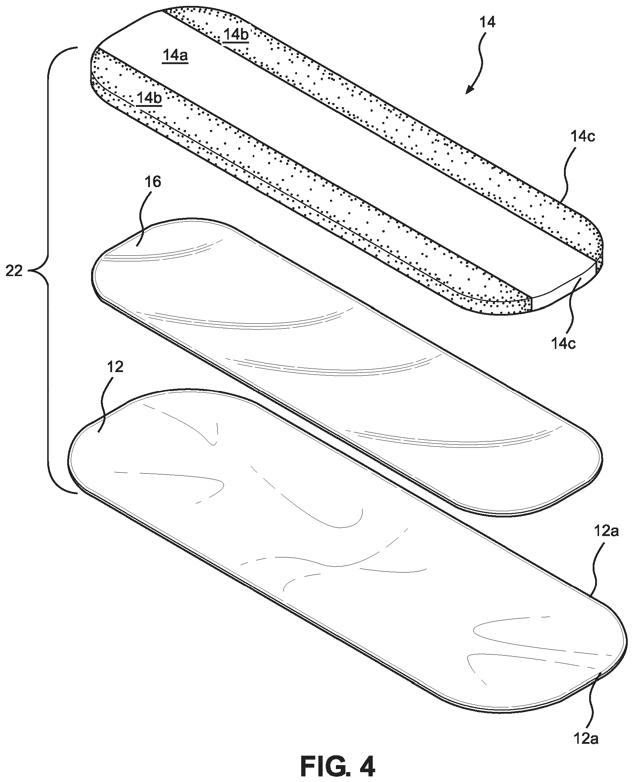

FIG. 4 is an exploded view of an island portion of the negative pressure wound dressing.
FIG. 5 is a top view of the island portion of the negative pressure wound dressing.

### DETAILED DESCRIPTION

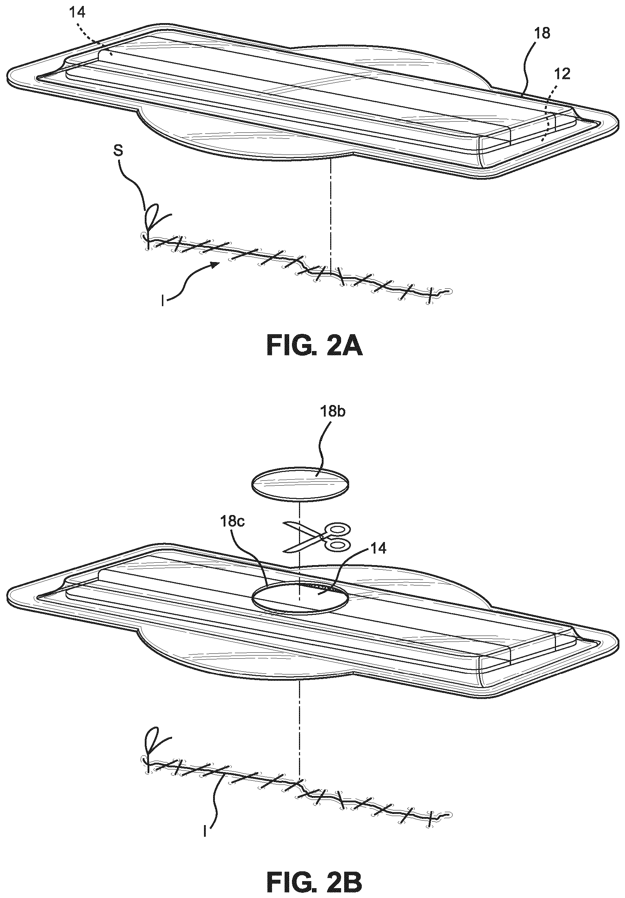

With reference to the drawings, the disclosure provides a negative pressure wound dressing **10** of improved construction. The dressing 10 is configured to transport wound fluids away more effectively from a wound, such as a surgical incision **I** such as a surgical incision skin **S** of a patient (FIG. 2A). It will be understood that the dressing 10 may be applied to wounds other than incisions.

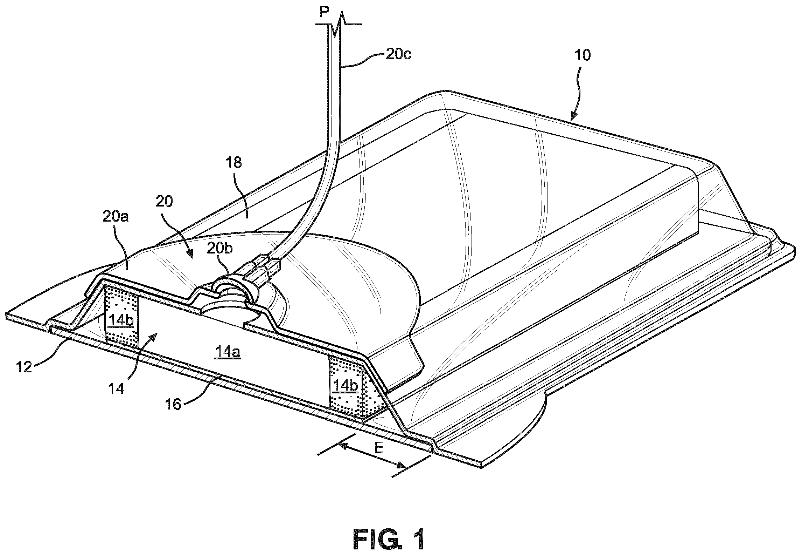

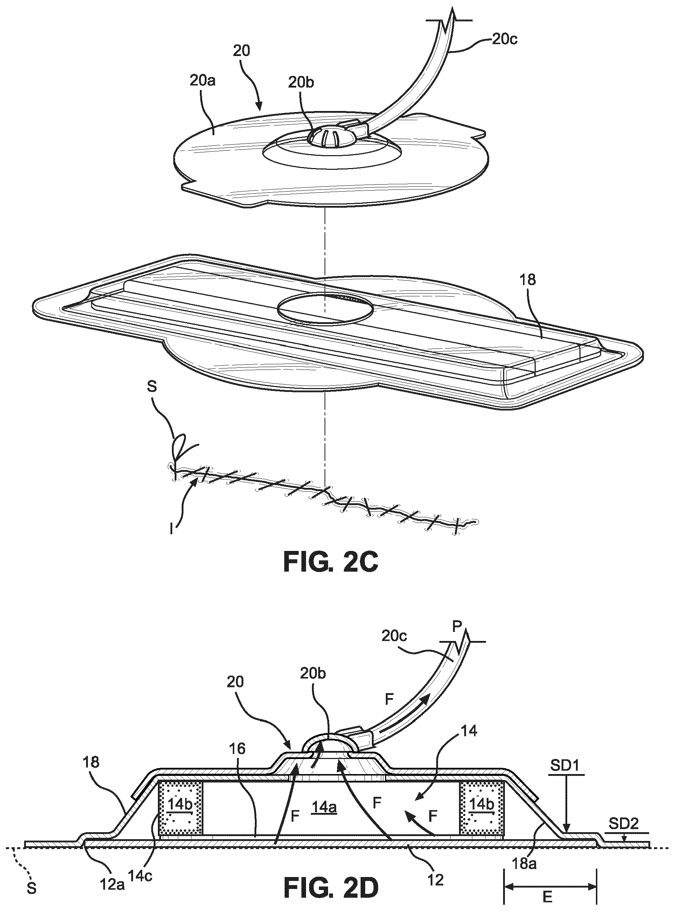

As shown in FIGS. 1 and 2, the dressing 10 includes a skin contact layer **12,** an absorbent layer **14,** an adhesive layer **16,** a negative pressure drape **18,** and a suction layer **20.** The skin contact layer adhered to the absorbent layer provides an island 22 component of the dressing 10.

A key feature of the dressing 10 is the configuration of the dressing 10 to help avoid blistering of skin associated with many conventional negative pressure wound dressings. As explained in more detail below, this is accomplished by providing the skin contact layer 12 of the dressing 10 to extend a distance beyond the periphery of the absorbent layer 14 of the dressing 10. This provides a more gradual step down of the drape 18 over the island 22 to the skin S at the juncture of the drape 18 and peripheral edges **12a** of the skin contact layer 12. This structure has been observed to reduce, if not avoid, blistering of skin at the juncture of the drape 18 and the edges 12a of the skin contact layer 12.

The skin contact layer 12 is a non-adherent wound contact material, preferably made of a non-adherent fluid permeable material such as polyester. Polyester is desirable to allow for both horizontal and vertical wicking of fluid from the incision I. The skin contact layer 12 has no adhesive on the bottom or skin facing surface thereof.

The absorbent layer 14 can be of unibody construction, but preferably includes an elongate liquid permeable center section **14a** and lateral absorbent sections **14b** that abut the center section 14a on each elongate side of the center section 14a and are joined thereto by the adhesive layer 16. The absorbent layer 14 is a continuous body and does not include any apertures or openings. As will be noted, the absorbent layer 14 has peripheral edges 14c that are interior to the peripheral edges 12a of the skin contact layer 12.

The liquid permeable center section 14a is preferably made of a hydrophobic, porous, and fluid permeable foam material having a thickness of at least about 0.25 inches (0.635 centimetres). Most preferably, this fluid permeable foam material of the center section 14a is a reticulated polyurethane foam with a pores per inch range of 40-50 (15 pores per centimetre to 20 pores per centimetre). The center section 14a may also be thicker than the lateral absorbent sections 14b for use of the dressing 10. The thickness of the center section 14a is selected to be sufficiently thick to ensure there is enough cross-sectional area when the foam is under vacuum to be able to transport fluid and negative pressure effectively.

The lateral absorbent sections 14b may be thinner than the center section 14a, with a thickness of about 0.04 inches (0.1016 centimetres). This thinness of the lateral absorbent sections 14b aids in conforming the dressing 10 to the site of the incision I. The lateral absorbent sections 14b are constructed from an absorbent cellulose fiber material configured to absorb and contain wound exudate and like fluids from the incision I. If desired, the lateral absorbent sections 14b may include lyocell fibers, which are cellulose fibers prepared from cellulose solutions in an organic solvent, and having enhanced absorbency as compared to non-lyocell cellulose fibers. The lateral absorbent sections 14b may each be provided as by 7.9 ounce per square yard (267.9 grams per square metre) lyocell pads, having an absorbent capacity of about 65 ounces per square yard (2204 grams per square metre).

The adhesive layer 16 is an adhesive of a type commonly used with wound dressing. The adhesive layer 16 secures the absorbent layer 14 to the portion of the skin contact layer 12 that directly underlies the absorbent layer 14. The adhesive layer 16 does not extend past the absorbent layer 14.

The negative pressure drape 18 may be provided by an adhesive polyurethane film. The drape 18 is semi occlusive and provides a substantially fluid-tight seal around the incision I so that a negative pressure can be applied to the incision 10. The drape 18 also provides a barrier at the top of the dressing 10 against fluid leakage from the dressing 10, and a barrier against bacteria to the incision 10 via the dressing 10. The moisture vapor transmission rate of the drape 18 is sufficient to be breathable to allow some fluid vapor to exit to avoid excess sweating of the wound, yet still be suitable for negative pressure purposes. A lowermost surface **18a** of the drape 18 is coated with an adhesive of a type commonly used with wound dressings so that the drape 18 adheres to the skin S. As shown, the drape 18 is generally rectangular, except desirably includes wings on the sides thereof for grasping to stretch the drape 18 during application and to provide additional securement surfaces.

The suction layer 20 may be of conventional construction to interface with the drape 18 and a conventional negative pressure or suction pump **P** to provide a negative or suction
pressure to the dressing 10. The pump P will usually feed any recovered fluids to a collection canister. As shown, the suction layer 20 includes a fluid barrier film **20a,** such as a polyurethane film, having adhesive on its lowermost surface. A fluid port **20b** is centrally provided to communicate with the underside of the film 20 for wound drainage fluids to communicate from the island 22 for withdrawal and for application of therapeutic pressure to the wound.

The port 20b connects to tubing **20c,** which connects to the negative pressure or suction pump P. The suction layer 20 may be a separate layer applied to the drape 18 as shown, or may be initially incorporated with the drape 18 during manufacture of the drape 18. A preferred suction device to provide the suction layer 20 is suction apparatus available from DeRoyal Industries, Inc. of Powell, Tennessee under the marks PROSPERA PROPEL and/or P-DOME.

Release liners **24a** and **24b** are secured to the bottom of the dressing 10 and secured in place by contact of upper surfaces of the liners 24a and 24b with adhesive of exposed portions of the lowermost surface 18a of the film 18 surrounding the center section 12 and the lateral absorbent sections 14. The release liners 24a and 24b may be conventional release liners used with bandages, typically being made of thin sheet paper with the surface in contact with the adhesive of the lowermost surface 18a being coated with a release coating such as silicone or other release coatings used for medical dressings.

A protective carrier film **26a** with a peeling tab **26b** is provided over the top of the dressing 10. Once the dressing 10 is applied, the carrier film 26a is removed using the peeling tab 26b.

FIGS. 2A-2D show one example of application of the dressing 10 to a wound site, such as the incision I. Initially, the island 22/drape 18 structure is aligned with the incision I and applied along the incision I. Next, the suction layer 20 is applied to the drape 18. If the dressing layer 20 is incorporated into the dressing 10 during manufacture, this step is not needed as the suction layer 20 is already present. In the depicted application of the suction layer 20 to the dressing 10, a cutout **18b** of the drape 18 is made and removed to provide an access aperture **18c** that exposes the absorbent layer 14. The suction layer 20 is applied over the aperture 18c to place the fluid port 20b at the location of the access aperture 18c to overlie the exposed portion of the absorbent layer 14 to create a pressure/fluid flow path between the absorbent layer 14 and the fluid port 20b. During use of the dressing 10, negative pressure applied via the pump P creates a flow of pressure/fluids generally depicted by arrows **F** corresponding to the negative pressure applied to the site of the wound by the pump P via the dressing 10.

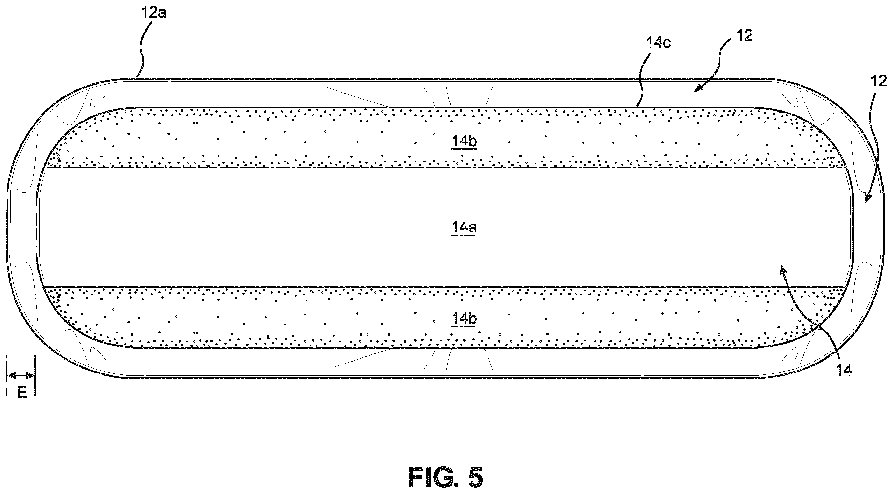

With specific reference to FIGS. 1, 2D and 5, the peripheral edges 12a of the skin contact layer 12 of the dressing 10 extend a distance **E** beyond the peripheral edges 14c of the absorbent layer 14 of the dressing 10. The distance E is preferably substantially uniform, but that is not necessarily required.

As will be noted, in the structure of the dressing 10, there is a step down **SD1** in height from the edge of the absorbent layer 14 to the skin contact layer 12, and a step down **SD2** in height from the skin contact layer 12 to the skin S of the patient. The location of the step down SD1 is spaced from the location of the step down SD2, and the step down SD2 at the edges 12a of the skin contact layer 12 is much smaller than the step down SD2.

Thus, the thickness of the island 12 is reduced at the edges 12a of the skin contact layer 12 as compared to other locations of the island 22. This provides a more gradual step down of the drape 18 over the island 22 to the skin S at the juncture of the drape 18 and the edges 12a of the skin contact layer 12. This structure has been observed to reduce, if not avoid, blistering of skin at the juncture of the drape 18 and the edges 12a of the skin contact layer 12, resulting from application of negative pressure to the dressing 10 for treatment of the incision I.

The dressing 10 is configured for incision wounds where the incision I has a length ranging from about 13cm (5.1 in) to about 35 cm (13.8in). Common dressing sizes for these incisions are (13 cm) 11.5 inches x 7.5 inches (29.21 centimetres x 19.05 centimetres); (20 cm/7.9in) 14.25 inches x 7.5 inches (36.195 centimetres x 19.05 centimetres); and (35 inches) 20.125 inches x 7.5 inches (51.1175 centimetres x 19.05 centimetres)). For this range of dressing sizes, it has been discovered that a practical range of the distance E is from a minimum of about 0.25 inches (0.635 centimetres) to a maximum of about 1 inch (2.54 centimetres).

The depicted dressing 10 is for a 20cm long incision and is sized 14.25 inches (I) x 7.5 inches (w) (36.195 centimetres x 19.05 centimetres). For this dressing, the absorbent layer 14 has a width/length of 3.0/10.25 inches (7.62/26.035 centimetres) and the skin contact layer 12 has a width/length of 3.75/11.0 inches (9.525/27.94 centimetres). The drape 18 has a width/length of 7.5/14.25 inches (19.05/36.195 centimetres). Thus, the distance E that the skin contact layer 12 extends beyond the absorbent layer 14 is about 0.375 inches (0.9525 centimetres) on all edges. The rectangular part of the drape 18 (excluding the wings extending from the width) has a width/length of 6.0/14.25 inches (15.24/36.195 centimetres) and extends past the skin contact layer 12 by a distance of at least about 1.0 inch (2.54 centimetres) to allow for sufficient surface area of adhesive to skin contact in order to prevent the entire dressing from dislodging under normal use. The width of the wings is about 0.75 inches (1.905 centimetres) each, rendering the 7.5 inch (19.05 centimetres) width.

In this regard, since the drape 18 adheres to the skin S but the skin contact layer 12 does not, increasing the distance E beyond 1 inch (2.54 centimetres) for the noted range of dressing sizes has been observed to substantially reduce the adhesive contact of the drape 18 and compromise the ability of the dressing 18 to stay in place. A distance E of less than about 0.25 inches (0.635 centimetres) for this range of dressing sizes has been observed to not have the beneficial properties described herein. While the drape 18 could be made larger, this results in an undesirably oversized drape 18 that is unwieldy to install and use.

It is believed that skin deformation from pressure applied to the skin can cause skin injuries such as blistering. For example, physical deformation of dermal and subdermal tissue from the pressure may lead to a restriction of blood flow in that area and subsequently causes tissue necrosis. It has been observed that negative pressure wound dressings which have a skin contact layer of the same dimensions as the absorbent layer and not having an extended skin contact layer can cause erythema, blistering and other damage of the skin at the juncture of skin drape and the skin where the drape steps down to the with a large step-down corresponding to the height of the island.

It has been observed that dressings according to the disclosure, which have the extended skin contact layer, spread out the step down of the drape 18 to the skin S. There is the initial large step down SD1, but the drape does not contact the skin S at such location. Spaced from this is the smaller step down SD2, where the drape 18 finally does contact the skin S. This structure has been observed to avoid or at least reduce incidences of skin damage. Use of dressings according to the disclosure have been observed to avoid or at
least substantially reduce damage to the skin at the juncture of the skin contact layer and the drape.

The foregoing description of preferred embodiments for this disclosure has been presented for purposes of illustration and description. It is not intended to be exhaustive or to limit the disclosure to the precise form disclosed. Obvious modifications or variations are possible in light of the above teachings. The embodiments are chosen and described in an effort to provide the best illustrations of the principles of the disclosure and its practical application, and to thereby enable one of ordinary skill in the art to utilize the disclosure in various embodiments and with various modifications as are suited to the particular use contemplated.

## Claims

1. A negative pressure wound dressing (10), comprising:
an island (22) comprising a fluid permeable non-adhesive skin contact layer (12) connected to a fluid absorbent layer (14), the absorbent layer (14) having peripheral edges (14c) and the skin contact layer (12) having peripheral edges (12a) extending past the peripheral edges (14c) of the absorbent layer (14) a distance (E), the skin contact layer (12) being positionable onto skin (S) of a patient surrounding a wound (I);
a negative pressure drape (18) overlying the island (22) and having an adhesive lower surface (18a) and peripheral edges extending past the peripheral edges (12a) of the skin contact layer (12); and
a suction layer (20) connected to the negative pressure drape (18) and in fluid communication with a source of negative pressure and the absorbent layer (14) to enable negative pressure to be applied through the absorbent layer (14) to the wound (I), wherein the drape (18) steps down from the peripheral edges (14c) of the fluid absorbent layer (14) at a first step down (SD1) and the drape (18) steps down from the peripheral edges (12a) of the skin contact layer (12) at a second step down (SD2) spaced outward from the first step down (SD1), with the second step down (SD2) having a height that is shorter than that of the first step down (SD1), the drape (18) being adhesively securable to the skin (S) of the patient surrounding the wound (I) at a juncture of the negative pressure drape (18) and the peripheral edges (12a) of the skin contact layer (12) defined by the second step down (SD2) to provide a drape/skin juncture to enable negative pressure to be applied to the wound (I) via the dressing (10), and the second step down (SD2) reduces damage to the skin (S) at the drape/skin juncture during application of negative pressure to the wound (I).

2. The negative pressure wound dressing (10) of claim 1, wherein the peripheral edges (12a) of the skin contact layer (12) extend a distance (E) beyond the peripheral edges (14c) of the absorbent layer (14) of from about 0.25 inches (6.35 millimetres) to about 1.0 inches (25.4 millimetres).

## Patentansprüche

1. Unterdruck-Wundverband (10), umfassend:
eine Insel (22), die eine fluiddurchlässige, nicht klebenden Hautkontaktschicht (12) umfasst, die mit einer Fluid absorbierenden Schicht (14) verbunden ist, wobei die absorbierende Schicht (14) umlaufende Ränder (14c) aufweist und die Hautkontaktschicht (12) umlaufende Ränder (12a) aufweist, die sich um einen Abstand (E) an den umlaufenden Rändern (14c) der absorbierenden Schicht (14) vorbei erstrecken, wobei die Hautkontaktschicht (12) auf die Haut (S) eines Patienten um eine Wunde (I) herum positionierbar ist;
eine Unterdruckabdeckung (18), welche die Insel (22) überdeckt und eine klebende untere Oberfläche (18a) und umlaufende Ränder aufweist, die sich an den umlaufenden Rändern (12a) der Hautkontaktschicht (12) vorbei erstrecken; und
eine Saugschicht (20), die mit der Unterdruckabdeckung (18) verbunden ist, und in strömungstechnischer Kommunikation mit einer Unterdruckquelle und der Absorptionsschicht (14) steht, um zu ermöglichen, dass Unterdruck durch die Absorptionsschicht (14) hindurch auf die Wunde (I) angelegt wird, wobei die Abdeckung (18) von den umlaufenden Rändern (14c) der Fluid absorbierenden Schicht (14) in einer ersten Stufe (SD1) abfällt, und die Abdeckung (18) von den umlaufenden Rändern (12a) der Hautkontaktschicht (12) in einer zweiten Stufe (SD2) abfällt, die von der ersten Stufe (SD1) nach außen beabstandet ist, wobei die zweite Stufe (SD2) eine Höhe aufweist, die geringer als jene der ersten Stufe (SD1) ist, die Abdeckung (18) an der die Wunde (I) umgebenden Haut (S) des Patienten an einer Verbindungsstelle zwischen der Unterdruckabdeckung (18) und den umlaufenden Rändern (12a) der Hautkontaktschicht (12) klebend befestigbar ist, die durch die zweite Stufe (SD2) definiert ist, um eine Abdeckungs-/Hautverbindungsstelle bereitzustellen, um zu ermöglichen, dass Unterdruck an die Wunde (I) über den Verband (10) angelegt wird, und die zweite Stufe (SD2) die Schädigung der Haut (S) an der Abdeckungs-/Hautverbindungsstelle beim Anlegen von Unterdruck auf die Wunde (I) reduziert.

2. Unterdruck-Wundverband (10) nach Anspruch 1, wobei sich die umlaufenden Ränder (12a) der Hautkontaktschicht (12) um einen Abstand (E) an den umlaufenden Rändern (14c) der Absorptionsschicht (14) von etwa 0,25 Zoll (6,35 Millimeter) bis etwa 1,0 Zoll (25,4 Millimeter) vorbei erstrecken.

## Revendications

1. Pansement à pression négative (10), comprenant :
un îlot (22) comprenant une couche de contact cutané non adhésive perméable aux fluides (12) reliée à une couche absorbante de fluides (14), la couche absorbante (14) présentant des bords périphériques (14c) et la couche de contact cutané (12) présentant des bords périphériques (12a) s'étendant au-delà des bords périphériques (14c) de la couche absorbante (14) d'une distance (E), la couche de contact cutané (12) pouvant être positionnée sur la peau (S) d'un patient entourant une plaie (I) ;
un champ opératoire à pression négative (18) recouvrant l'îlot (22) et présentant une surface inférieure adhésive (18a) et des bords périphériques s'étendant au-delà des bords périphériques (12a) de la couche de contact cutané (12) ; et
une couche d'aspiration (20) reliée au champ opératoire à pression négative (18) et en communication fluidique avec une source de pression négative et la couche absorbante (14) pour permettre d'appliquer une pression négative à travers la couche absorbante (14) sur la plaie (I), dans lequel le champ opératoire (18) présente un premier décalage (SD1) par rapport aux bords périphériques (14c) de la couche absorbante de fluides (14) et le champ opératoire (18) présente un second décalage (SD2) par rapport aux bords périphériques (12a) de la couche de contact cutané (12) espacé vers l'extérieur du premier décalage (SD1), le second décalage (SD2) présentant une hauteur plus courte que celle du premier décalage (SD1), le champ opératoire (18) pouvant être fixé par adhésif à la peau (S) du patient autour de la plaie (I) au niveau d'une jonction entre le champ opératoire à pression négative (18) et les bords périphériques (12a) de la couche de contact cutané (12), la jonction étant définie par le second décalage (SD2) pour fournir une jonction champ opératoire/peau permettant d'appliquer une pression négative à la plaie (I) via le pansement (10), et le second décalage (SD2) réduit les lésions cutanées (S) au niveau de la jonction champ opératoire/peau lors de l'application d'une pression négative sur la plaie (I).

2. Pansement à pression négative (10) selon la revendication 1, dans lequel les bords périphériques (12a) de la couche de contact cutané (12) s'étendent d'une distance (E) au-delà des bords périphériques (14c) de la couche absorbante (14) d'environ 0,25 pouce (6,35 millimètres) à environ 1,0 pouce (25,4 millimètres).
